# EUROPEAN PATENT APPLICATION

(11) **EP 4 454 643 A1**
(43) Date of publication of application: **30.10.2024**
(21) Application number: 22911257.8
(22) Date of filing: 21.12.2022
(51) Int. Cl.: A61K 31/122, A61K 47/14, A61K 47/36, A61P 9/00, A61P 17/18, A61P 39/06, A61P 43/00, C07C 46/10, C07C 50/06

(54) **METHOD FOR STORING REDUCED COENZYME Q10**

(30) Priority: 24.12.2021 JP 2021210433; 26.09.2022 JP 2022152253
(71) Applicant: Kaneka Corporation, Osaka-shi, Osaka 530-8288 (JP)
(72) Inventor: YOKOCHI Yuichi, Takasago-shi, Hyogo 676-8688 (JP); KITAMURA Shiro, Osaka-shi, Osaka 530-8288 (JP); FUKUYAMA Yuka, Takasago-shi, Hyogo 676-8688 (JP)
(74) Representative: Hoffmann Eitle
(86) International application number: PCT/JP2022/047018
(87) International publication number: WO 2023/120555

(57) **Abstract**

The present description discloses a preferred solution for controlling oxidation of QH without any need for formulation of reduced coenzyme Q10 (QH). One or more embodiments of the present invention relate to a method for storing reduced coenzyme Q10 (QH) or a method for controlling oxidation of QH, the method(s) including storing a composition containing a reduced coenzyme Q10 (QH) and having a water activity at 25°C of 0.50 or more. One or more other embodiments of the present invention relate to a composition containing QH and having a water activity at 25°C of 0.50 or more.

## Description

### Technical Field

One or more embodiments of the present invention relate to a method for storing reduced coenzyme Q10, a method for controlling oxidation of reduced coenzyme Q10, and a composition comprising reduced coenzyme Q10.

### Background Art

Coenzyme Q is an essential component widely distributed in living organisms from bacteria to mammals, and is known as a member of mitochondrial electron transfer system in cells in the living organisms. The major component in humans is coenzyme Q10 which is one having 10 repeating structures in the side chain of coenzyme Q, and generally, about 40% to 90% thereof is present in the living body as the reduced form. The physiological activity of coenzyme Q includes activation of energy production by mitochondrial activation, activation of cardiac function, stabilization of cell membranes, and protection of cells by antioxidant activity.

While coenzyme Q10 currently produced and sold is, in large part, oxidized coenzyme Q10, reduced coenzyme Q10 (hereinafter sometimes referred to as "QH") which exhibits higher oral absorbability than that of oxidized coenzyme Q10 has also been commercially available and has come to be used in recent years.

Reduced coenzyme Q10 is easily oxidized, which leads to a problem that the storage cost is high and the scope of application of commercial products is restricted.

A common method for obtaining reduced coenzyme Q10 has already been disclosed (Patent Literature 1). Patent Literature 2 reports that crystal polymorphism is found in reduced coenzyme Q10. Patent Literature 2 reports that a newly appearing crystal form (wherein this crystal is hereinafter referred to as a "reduced coenzyme Q10 Form II crystal" or "QH Form II crystal") is much more stable than the conventional reduced coenzyme Q10 (wherein this crystal is hereinafter referred to as a "reduced coenzyme Q10 Form I crystal" or "QH Form I crystal") and also has other excellent physical properties.

Examples of the literature disclosing a technique for controlling oxidation of reduced coenzyme Q10 and stably storing reduced coenzyme Q10 include Patent Literature 3, Patent Literature 4 and Patent Literature 5. These Patent Literature describe, as reduced coenzyme Q10 having high oxidation stability and high *in vivo* absorbability, a particulate composition in which an oily component containing reduced coenzyme Q10 or an oily component containing reduced coenzyme Q10 and a lipophilic antioxidant forms a domain and is poly-dispersed in a matrix containing a water-soluble excipient or a matrix containing a water-soluble excipient and water-soluble ascorbic acids, and describe a method for stabilizing the particulate composition by placing the particulate composition in an ambient environment having a relative humidity of 90% or less. Examples of the water-soluble excipient described include gum arabic and gelatin. All of Patent Literature 3 to 5 disclose a technique for increasing oxidation stability by coating QH with a coating film of a gas barrier material such as gum arabic or gelatin.

Organic compounds are known to be generally less stable at a higher relative humidity (Non-Patent Literature 1 and 2).

Patent Literature 6 describes a co-crystal containing reduced coenzyme Q10 and a compound such as 3,4-dihydroxybenzoic acid, which has been found as an additional form of reduced coenzyme Q10. Patent Literature 7 describes formation of a co-crystal of reduced coenzyme Q10 and nicotinamide. Although reduced coenzyme Q10 may have increased oxidation stability due to co-crystallization of reduced coenzyme Q10 and one or more other compounds, even such co-crystallization cannot completely prevent reduced coenzyme Q10 from being oxidized.

### Citation List

### Patent Literature

Patent Literature 1: JP Patent Publication No. H10-109933
Patent Literature 2: WO2012/176842
Patent Literature 3: WO2007/148798
Patent Literature 4: WO2008/129980
Patent Literature 5: JP Patent Publication No. 2009-149584 A
Patent Literature 6: WO2019/162429
Patent Literature 7: Chinese Patent Publication No. 113024362 A

### Non-Patent Literature

Non-Patent Literature 1: Evaluating Stability of Vitamin C in Fortified Formula Using Water Activity and Glass Transition, Sablani SS, Al-Belushi K, Al-Marhubi I, and Al-Belushi R, International Journal of Food Properties, 2007, 10(1):61-71
Non-Patent Literature 2: Preformulation Studies of a Prodrug of Δ9-Tetrahydrocannabinol, Thumma S, Majumdar S, ElSohly MA, Gul W, and Repka MA, 2008, AAPS Pharm Sci Tech, 9(3):982-990

### Summary of Invention

### Technical Problem

Patent Literature 3 to 5 disclose a QH product that prevents oxidation of reduced coenzyme Q10 (QH) and can be stably stored. However, QH is required to be formulated with a specified component in all of Patent Literature 3 to 5, and thus applications of QH are limited.

One or more embodiments of the present invention provide a method for storing QH, a method for controlling oxidation of QH, and a composition containing QH, which are capable of controlling oxidation of QH without requiring formulation of QH.

### Solution to Problem

As described above, organic compounds are generally less stable at a higher humidity. Meanwhile, the present inventors have unexpectedly found that QH has high oxidation stability in a composition having high water activity, and have completed the following respective aspects of the present invention.
(1) A method for storing reduced coenzyme Q10, including
   storing a composition containing reduced coenzyme Q10 and having a water activity at 25°C of 0.50 or more.
(2) The method according to (1), wherein the composition is a mixed composition in which the reduced coenzyme Q10 and one or more other components are mixed.
(3) The method according to (1), wherein the composition is a multiphase composition including a first phase containing the reduced coenzyme Q10 and a second phase containing one or more other components, the first and second phases being in contact with each other.
(4) The method according to any one of (1) to (3), wherein the reduced coenzyme Q10 is one or more selected from the group consisting of a reduced coenzyme Q10 Form I crystal, a reduced coenzyme Q10 Form II crystal, a co-crystal composed of reduced coenzyme Q10 and one or more other compounds, an amorphous solid of reduced coenzyme Q10, and a composition in which reduced coenzyme Q10 is dissolved in a solvent and/or a fat-soluble material.
(5) The method according to any one of (1) to (4), wherein the composition includes a substance releasing water.
(6) A method for controlling oxidation of reduced coenzyme Q10, including
   storing a composition containing reduced coenzyme Q10 and having a water activity at 25°C of 0.50 or more.
(7) The method according to (6), wherein the composition is a mixed composition in which the reduced coenzyme Q10 and one or more other components are mixed.
(8) The method according to (6), wherein the composition is a multiphase composition including a first phase containing the reduced coenzyme Q10 and a second phase containing one or more other components, the first and second phases being in contact with each other.
(9) The method according to any one of (6) to (8), wherein the reduced coenzyme Q10 is one or more selected from the group consisting of a reduced coenzyme Q10 Form I crystal, a reduced coenzyme Q10 Form II crystal, a co-crystal composed of reduced coenzyme Q10 and one or more other compounds, an amorphous solid of reduced coenzyme Q10, and a composition in which reduced coenzyme Q10 is dissolved in a solvent and/or a fat-soluble material.
(10) The method according to any one of (6) to (9), wherein the composition includes a substance releasing water.
(11) A composition including reduced coenzyme Q10 and having a water activity at 25°C of 0.50 or more.
(12) The composition according to (11), wherein the composition is a mixed composition in which the reduced coenzyme Q10 and one or more other components are mixed.
(13) The composition according to (11), wherein the composition is a multiphase composition including a first phase containing the reduced coenzyme Q10 and a second phase containing one or more other components, the first and second phases being in contact with each other.
(14) The composition according to any one of (11) to (13), including a substance releasing water.

The present description encompasses the specifications and/or drawings in JP Patent Application Nos. 2021-210433 and 2022-152253 which serve as the basis of the priority of the present application.

### Advantageous Effects of Invention

According to the method for storing reduced coenzyme Q10 (QH) disclosed herein, oxidation of QH can be controlled and QH can be stably stored.

According to the method for controlling oxidation of QH disclosed herein, oxidation of QH can be effectively controlled.

According to the composition containing QH disclosed herein, oxidation of QH can be controlled and QH can be stably stored.

### Description of Embodiments

Hereinafter, the present invention will be described in detail.

### <Reduced Coenzyme Q10>

"Reduced coenzyme Q10" in one or more embodiments of the present invention may partially include oxidized coenzyme Q10, provided that reduced coenzyme Q10 is included as a main component. The "main component" herein means that it is included in a percentage of, for example, 50% by weight or more, generally 60% by weight or more, preferably 70% by weight or more, more preferably 80% by weight or more, further preferably 90% by weight or more, particularly preferably 95% by weight or more, and further particularly 98% by weight or more. Herein, the above-described percentages are the percentages of reduced coenzyme Q10 to the total weight of coenzyme Q10.

Besides, as mentioned above, reduced coenzyme Q10 has two forms of crystal polymorphisms, namely, Form I and Form II. Specifically, the crystal form of reduced coenzyme Q10 having a melting point around 48°C and showing characteristic peaks at diffraction angles (2θ ± 0.2°) of 3.1°, 18.7°, 19.0°, 20.2°, and 23.0° in powder X-ray (Cu-Kα) diffraction is a Form I crystal, whereas the crystal form of reduced coenzyme Q10 having a melting point around 52°C and showing characteristic peaks at diffraction angles (2θ ± 0.2°) of 11.5°, 18.2°, 19.3°, 22.3°, 23.0°, and 33.3° in powder X-ray (Cu-Kα) diffraction is a Form II crystal.

One or more selected from the group consisting of a QH Form I crystal, a QH Form II crystal, a co-crystal composed of reduced coenzyme Q10 and one or more other compounds, an amorphous solid of QH, and a composition in which QH is dissolved in a solvent and/or a fat-soluble material can be used as the reduced coenzyme Q10 (QH) in one or more embodiments of the present invention. The solvent is not particularly limited as long as the solvent can dissolve QH. Examples of the solvent include alcohols, hydrocarbons, ketones, terpenes, fats and oils, essential oils, and propylene glycol fatty acid esters. The fat-soluble material is not particularly limited as long as the fat-soluble material can dissolve QH. Examples of the fat-soluble material may include fatty acids, an emulsifiers, fat-soluble vitamins, and derivatives of vitamins. At a temperature at which a method according to one or more embodiments of the present invention is carried out or a composition according to one or more embodiments of the present invention is stored or used, the composition in which QH is dissolved in the solvent and/or the fat-soluble material may be a liquid composition or a solid composition, and is preferably a liquid composition.

In the co-crystal composed of QH and one or more other compounds, such one or more other compounds included are not particularly limited as long as such each compound can form the co-crystal with QH, and examples thereof include organic carboxylic acids such as benzoic acid and derivatives thereof, organic alcohols such as resorcinol, benzyl alcohol and phenol, and derivatives thereof, urea, and nicotinamide. Such one or more other compounds may be one compound or two or more compounds, and are preferably one to three compounds.

The alcohols may be cyclic or acyclic and may be saturated or unsaturated, and are not particularly limited. Generally, examples thereof include those having 1 to 20 carbon atoms, preferably those having 1 to 12 carbon atoms, further preferably those having 1 to 5 carbon atoms, further particularly preferably those having 1 to 4 carbon atoms, and in particular, preferably a monohydric alcohol, most preferably a monohydric alcohol having 2 carbon atoms. A dihydric alcohol having 2 to 5 carbon atoms, preferably 2 to 4 carbon atoms, further preferably 3 carbon atoms, or a trihydric alcohol having 3 carbon atoms is also preferably used.

Examples of the monohydric alcohol may include methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol, 1-hexanol, 2-methyl-1-pentanol, 4-methyl-2-pentanol, 2-ethyl-1-butanol, 1-heptanol, 2-heptanol, 3-heptanol, 1-octanol, 2-octanol, 2-ethyl-1-hexanol, 1-nonanol, 1-decanol, 1-undecanol, 1-dodecanol, allyl alcohol, propargyl alcohol, benzyl alcohol, cyclohexanol, 1-methylcyclohexanol, 2-methylcyclohexanol, 3-methylcyclohexanol, 4-methylcyclohexanol, cinnamyl alcohol, phenol, and α-methylbenzyl alcohol, and the monohydric alcohol is most preferably ethanol.

Examples of the dihydric alcohol may include 1,2-ethanediol, 1,2-propanediol (propylene glycol), 1,3-propanediol, 1,2-butanediol, 1,3-butanediol, 1,4-butanediol, 2,3-butanediol, and 1,5-pentanediol. The dihydric alcohol is preferably 1,2-ethanediol, 1,2-propanediol, 1,3-butanediol, or 1,3-propanediol, and most preferably, 1,2-propanediol.

Glycerin or the like can be preferably used as the trihydric alcohol.

The hydrocarbons are not particularly limited, and examples thereof may include aliphatic hydrocarbon, aromatic hydrocarbon, and halogenated hydrocarbon. In particular, aliphatic hydrocarbon and aromatic hydrocarbon are preferable, and aliphatic hydrocarbon is further particularly preferable.

The aliphatic hydrocarbon may be cyclic or acyclic and may be saturated or unsaturated, and are not particularly limited, and acyclic aliphatic hydrocarbon is particularly preferably used. In general, one having 3 to 20 carbon atoms, preferably 5 to 12 carbon atoms is used.

Examples of the aliphatic hydrocarbon may include propane, butane, isobutane, pentane, 2-methylbutane, cyclopentane, 2-pentene, hexane, 2-methylpentane, 2,2-dimethylbutane, 2,3-dimethylbutane, methylcyclopentane, cyclohexane, 1-hexene, cyclohexene, heptane, 2-methylhexane, 3-methylhexane, 2,3-dimethylpentane, 2,4-dimethylpentane, methylcyclohexane, 1-heptene, octane, 2,2,3-trimethylpentane, isooctane, ethylcyclohexane, 1-octene, nonane, 2,2,5-trimethylhexane, 1-nonene, decane, 1-decene, p-menthane, undecane, and dodecane.

The ketones may be cyclic or acyclic and may be saturated or unsaturated, and are not particularly limited. Examples thereof may include acetone, methyl ethyl ketone, methyl butyl ketone, methyl isobutyl ketone, acetophenone, 4-methoxyphenylacetone, para-methylacetophenone, and methyl β-naphthyl ketone. The ketones are preferably acetone, acetophenone, 4-methoxyphenylacetone, para-methylacetophenone, and/or methyl ethyl ketone.

The terpenes are not particularly limited, and any of hemiterpene, monoterpene, sesquiterpene, diterpene, sesterterpene, and/or triterpene can be preferably used. In particular, hemiterpene, monoterpene, and sesquiterpene are more preferable, monoterpene and sesquiterpene are particularly preferable, and monoterpene is most preferable from the viewpoint of solubility of QH.

Examples of the terpenes may include prenol, 3-methyl-3-buten-2-ol, tiglic acid, angelic acid, senecioic acid, isovaleric acid, allo-ocimene, β-bourbonene, δ-cadinene, dehydro-p-cymene, menthol, dl-limonene, d-limonene, 1-limonene, p-cymene, α-pinene, valencene, myrcene, bisabolene, carene, caryophyllene, terpinene, phytol, cis-3,7-dimethyl-1,3,6-octatriene, δ-elemene, β-elemene, α-farnesene, β-farnesene, farnesene, germacrene D, β-guaiene, longifolene, β-ocimene, α-phellandrene, pinocamphone, sabinene, terpinolene, thujopsene, α-copaene, hydrogenated limonene dimer, isocaryophyllene, pinene dimer, dipentene dimer, dipentene trimer, geraniol, citral, citronellal, citronellol, 1,8-cineol, hydroxycitronellal, linalool, cosmene, nerol, myrcenol, lavandulol, ipsdienol, neral, geranial, perylene, rose furan, geranyl acid, thioterpineol, α-terpineol, β-terpineol, γ-terpineol, δ-terpineol, carveol, terpin, perillaldehyde, perillalcohol, carvone, ascaridole, anethole, thujone, thujanol,, α-ionone, β-ionone, γ-ionone, farnesol, nerolidol, α-sinensal, β-sinensal, bisabol, squalene, citronellyl oxyacetoaldehyde, myrtenal, perillaldehyde, 2-p-cymenol, 2-ethoxy-p-cymene, carvenol, 4-carvomenthenol, carvyl acetate, carvyl propionate, caryophyllene alcohol, caryophyllene alcohol acetate, 1,4-cineol, eugenol, d-selinene, thymol, d-camphene, and linalool acetate. The terpenes are most preferably dl-limonene and/or d-limonene.

The fat and oil may be natural fat and oil from animal or plant, synthetic fat and oil, or processed fat and oil. Examples of plant fat and oil may include coconut oil, palm oil, palm kernel oil, flaxseed oil, camellia oil, brown rice germ oil, rape seed oil, rice oil, peanut oil, corn oil, wheat germ oil, soybean oil, perilla oil, cottonseed oil, sunflower seed oil, kapok oil, evening primrose oil, Shea butter, Sal butter, cacao butter, sesame oil, safflower oil, olive oil, avocado oil, poppy oil, and arctium lappa seed oil, and examples of animal fat and oil may include lard, milk fat, fish oil, and beef fat, and may also further include fat and oil (e.g., hardened oil) obtained by processing the above by fractionation, hydrogenation, transesterification, and/or the like. Needless to say, medium-chain fatty acid triglyceride (MCT), partially glyceride of fatty acid, or the like can also be used. A mixture thereof may also be used.

The medium-chain fatty acid triglyceride is not particularly limited, and examples thereof may include triglyceride in which each fatty acid has 6 to 12, preferably 8 to 12 carbon atoms.

The essential oil is not particularly limited and is preferably essential oil containing terpenes, and examples thereof may include orange oil, capsicum oil, mustard oil, garlic oil, caraway oil, clove oil, cinnamon oil, cocoa extract, coffee bean extract, ginger oil, spearmint oil, celery-seed oil, thyme oil, onion oil, nutmeg oil, parsley seed oil, mint oil, vanilla extract, funnel oil, pennyroyal oil, peppermint oil, eucalyptus oil, lemon oil, rose oil, rosemary oil, almond oil, ajowan oil, anise oil, amyris oil, angelica root oil, ambrette seed oil, estragon oil, origanum oil, orris root oil, olibanum oil, cassia oil, cascarilla oil, cananga oil, chamomile oil, calamus oil, cardamom oil, carrot seed oil, cubeb oil, cumin oil, grapefruit oil, cinnamon leaf oil, cade oil, pepper oil, costus root oil, cognac oil, copaiba oil, coriander oil, perilla oil, musk oil, juniper berry oil, star anise oil, sage oil, savory oil, geranium oil, tangerine oil, dill oil, neroli oil, tolu balsam oil, basil oil, birch oil, patchouli oil, palmarosa oil, pimento oil, petitgrain oil, bay leaf oil, bergamot oil, peru balsam oil, benzoin resin, Bois de Rose oil, hop oil, boronia absolute, marjoram oil, mandarin oil, myrtle oil, citrus junos flavor, lime oil, lavandin oil, lavender oil, rue oil, lemongrass oil, lenthionine, lavage oil, laurel leaf oil, and worm wood oil.

The propylene glycol fatty acid ester is not particularly limited, and examples thereof may include propylene glycol monocaprylate, propylene glycol dicaprylate, propylene glycol monocaprate, propylene glycol dicaprate, propylene glycol monolaurate, propylene glycol dilaurate, propylene glycol monomyristate, propylene glycol dimyristate, propylene glycol monopalmitate, propylene glycol dipalmitate, propylene glycol monostearate, propylene glycol distearate, propylene glycol monoisostearate, propylene glycol diisostearate, propylene glycol monooleate, propylene glycol dioleate, propylene glycol monolinoleate, propylene glycol dilinoleate, propylene glycol monolinolenate, and propylene glycol dilinolenate. Besides the above, a propylene glycol difatty acid ester in which two fatty acid residues are different may also be adopted.

Examples of the fatty acid may include caprylic acid, capric acid, lauric acid, myristic acid, palmitic acid, stearic acid, isostearic acid, oleic acid, linoleic acid, and linolenic acid.

Examples of the emulsifier may include glycerin fatty acid esters, sucrose fatty acid esters, sorbitan fatty acid esters, polyglycerin-condensed ricinoleic acid esters, polyoxyethylene sorbitan fatty acid esters, saponins, and phospholipid.

The phospholipid is not particularly limited, and examples thereof may include lecithin such as egg-yolk lecithin and purified soybean lecithin, phosphatidylcholine, phosphatidylethanolamine, phosphatidylserine, sphingomyelin, dicetyl phosphoric acid, stearylamine, phosphatidylglycerol, phosphatidic acid, phosphatidylinositolamine, cardiolipin, ceramide phosphoryl ethanolamine, ceramide phosphoryl glycerol, and any mixture thereof. Any phospholipid subjected to processing such as hydrogenation or enzyme degradation (e.g., hydrogenated lecithin or lysolecithin) can also be used.

Examples of the fat-soluble vitamins may include vitamin E, vitamin A, vitamin D, and vitamin K.

Examples of the derivatives of vitamins may include derivatives of the fat-soluble vitamins, and fat-soluble derivatives obtained by modifying water-soluble vitamins to fat-soluble. Examples of the water-soluble vitamins may include vitamin C, vitamin B1, vitamin B2, vitamin B6, vitamin B12, niacin, pantothenic acid, folic acid, and biotin.

QH used in one or more embodiments of the present invention is not required to be pre-formulated (i.e., need not be formulated in advance). It is preferable that QH in one or more embodiments of the present invention consists of non-preformulated QH, such as one or more selected from the group consisting of a QH Form I crystal, a QH Form II crystal, a co-crystal composed of QH and one or more other compounds, an amorphous solid of QH, and a composition in which QH is dissolved in a solvent and/or a fat-soluble material, because in such a case QH can be utilized in a wide range of applications. More preferably, QH is not pre-formulated QH (e.g., a clathrate of QH with cyclodextrin, QH dispersed in a matrix containing a water-soluble excipient in a particulate composition, QH coated with a coating material in a solid formulation, or a capsule of QH).

The water-soluble excipient can be, for example, one or more selected from the group consisting of a water-soluble polymer, a surfactant, sugar, and a yeast cell wall.

The coating material can be, for example, an oil-soluble coating material or a water-soluble coating material. The oil-soluble coating material can be, for example, a sugar ester of a higher fatty acid, shellac, a cellulose derivative, fatty acids and ester derivatives thereof, fats and oils, or zein. The water-soluble coating material can be, for example, gelatin, sugar, gum arabic, a sugar ester of a higher fatty acid, tragacanth, pectin, pullulan, alginic acid, dried albumen, milk, curdlan, a cellulose derivative, casein, a casein compound, starch, or a yeast cell wall.

The capsule is, for example, QH capsulated by a soft capsule, a hard capsule, a microcapsule, or the like. Examples of the material of the capsule may include gelatin derived from beef bone, bovine hide, pig hide, fishskin, or the like; a seaweed-derived product usable as a food additive, such as carrageenan or alginic acid; a plant seed-derived product such as locust bean gum or guar gum; an agent for production, containing cellulose; and starch such as wheat starch, potato starch, sweet potato starch, corn starch, or dextrin.

### <Method for Storing QH>

A first embodiment of the present invention relates to a method for storing QH, including storing a composition containing QH and having a water activity at 25°C of 0.50 or more.

According to the method according to the present embodiment, oxidation of QH can be controlled and QH can be stably stored. In general, compounds are considered unstable in the presence of water, and it is considered important to store them in a dry state. The method according to the present embodiment utilizes the property that QH is stabilized in the state of being contained in a composition having water activity of 0.50 or more at 25°C, which is unique to QH and contrary to the above common belief to allow QH to be stably stored. Preparation of the composition is easily carried out. Therefore, the method according to the present embodiment can serve as a measure for efficiently stabilizing QH at a low cost.

QH in the form of a QH Form II crystal, a co-crystal composed of QH and one or more other compounds, or a composition in which QH is dissolved in a solvent and/or a fat-soluble material is itself costly to produce, and therefore a QH residual rate after storage (refer to the definition in Examples) of 90% or more is desired to provide QH in the above form at an appropriate price. The method according to the present embodiment is preferable in that the QH residual rate after storage can be 90% or more when QH is in the form of a QH Form II crystal, a co-crystal composed of QH and one or more other compounds, or a composition in which QH is dissolved in a solvent and/or a fat-soluble material.

QH in the form of a QH Form I crystal can be produced at a low cost, but is easily oxidized. Therefore, it is desired that the QH residual rate after storage (refer to the definition in Examples) is 40% or more, in order to provide QH in the above form at an appropriate price. The method according to the present embodiment is preferable in that the QH residual rate after storage can be 40% or more when QH is in the form of a QH Form I crystal.

The composition containing QH and having a water activity at 25°C of 0.50 or more in the method according to the present embodiment will be described below.

The water activity of the composition can be measured by an ordinary method.

The water activity at 25°C of the composition is preferably 0.53 or more, more preferably 0.60 or more, further preferably 0.70 or more, further preferably 0.75 or more, further preferably 0.80 or more, further preferably 0.85 or more, and particularly preferably 0.90 or more. In particular, when QH is a Form I crystal, the water activity is preferably 0.53 or more, more preferably 0.60 or more, further preferably 0.70 or more, further preferably 0.75 or more, further preferably 0.80 or more, further preferably 0.85 or more, and particularly preferably 0.90 or more. When QH is other than a Form I crystal, the water activity is preferably 0.53 or more, more preferably 0.60 or more, particularly preferably 0.70 or more, and most preferably 0.75 or more.

The composition may include one or more other components in addition to QH. The one or more other components may be any component usable in combination with QH, and examples thereof include a component acceptable as a food, a cosmetic product, or a pharmaceutical product. When at least one of a co-crystal composed of QH and one or more other compounds, and a composition in which QH is dissolved in a solvent and/or a fat-soluble material is used as QH in the composition, the one or more other components are contained in addition to the co-crystal, the solvent and/or the fat-soluble material.

The one or more other components is preferably one or more components containing water, or a substance releasing water, in order to control the water activity of the composition to 0.50 or more at 25°C. Examples of the one or more components containing water may herein include water, or one or more components containing water, selected from carbohydrate, sugar, protein, lipid, salts, and the like. The water means water present as a liquid, is not necessarily needed to be pure water, and may be present as an aqueous solution. The aqueous solution may be an aqueous solution of a salt in which an inorganic salt is dissolved in water, in order to control the water activity to less than 1.0. The substance releasing water is a substance which gradually releases water vapor.

In a preferred aspect, the composition is a mixed composition in which QH and one or more other components are mixed. The mixed composition can be a composition acceptable as a food, a cosmetic product, or a pharmaceutical product, as a whole.

The mixed composition may be a mixed composition in which QH and one or more other components are homogeneously mixed, or may be a mixed composition in which QH and one or more other components are heterogeneously mixed. Such a mixed composition homogeneously mixed refers to a composition containing QH and one or more other components, in which the concentration distribution of QH in the entire composition is homogeneous or substantially homogeneous. Such a mixed composition homogeneously mixed can be obtained by, for example, sufficiently mixing QH and one or more other components. Such a mixed composition heterogeneously mixed refers to a composition containing QH and one or more other components, in which the concentration distribution of QH is not homogeneous and is biased. Such a mixed composition heterogeneously mixed can be obtained by, for example, adding QH to one or more other components such as a material for foods.

In another preferred aspect, the composition is a multiphase composition including a first phase containing QH and a second phase containing one or more other components, in which the phases are in contact with each other. The multiphase composition can be a composition acceptable as a food, a cosmetic product, or a pharmaceutical product, as a whole, or at least the first phase can be a composition acceptable as a food, a cosmetic product, or a pharmaceutical product when the first phase and the second phase can be separated. The first phase refers to a phase composed of QH or a homogeneous phase containing QH, where the QH contains, for example, one or more selected from the group consisting of a QH Form I crystal, a QH Form II crystal, a co-crystal composed of QH and one or more other compounds, an amorphous solid of QH, and a composition in which QH is dissolved in a solvent and/or a fat-soluble material. The second phase refers to a phase which can be in contact with the first phase without being mixed with the first phase. The multiphase composition can be, for example, one in which the first phase and the second phase are stacked, or one in which one of the first phase and the second phase is supported on the other. One example of the multiphase composition is one including a first phase composed of a composition in which QH is dissolved in a solvent and/or a fat-soluble material, and a second phase composed of water or an aqueous solution immiscible with the first phase, in which one of the first phase and the second phase is stacked on the other. Another example of the multiphase composition is one including a first phase composed of QH-containing particles and a second phase composed of one or more other components in matrix form, in which the first phase is supported on the second phase. Still another example of the multiphase composition is one including a first phase containing QH and a second phase containing a substance releasing water, in which the first phase and the second phase are in contact with each other.

The content of QH in the composition is not particularly limited. The content of QH based on the total mass of the composition can be, for example, 0.01% by mass or more, preferably 0.1% by mass or more, and more preferably 1% by mass or more, and can be, for example, 99% by mass or less, preferably 90% by mass or less, and more preferably 50% by mass or less.

The temperature at which the composition is stored in the method according to the present embodiment is, for example, a temperature of -25°C or more and 50°C or less, preferably a temperature of -20°C or more, -10°C or more, 0°C or more, 4°C or more, 10°C or more, 15°C or more, 20°C or more, or 25°C or more, and preferably a temperature of 45°C or less, or 40°C or less. The temperature can be specifically 25°C or 40°C.

The period for which the composition is stored in the method according to the present embodiment is not particularly limited as long as it is a period until use of a product after production, and can be appropriately adjusted depending on storage conditions such as temperature. The period is preferably 3 days or more, 1 week or more or 2 weeks or more and can be, for example, 5 years or less, generally 3 years or less, preferably 2 years or less, more preferably 1 year or less, further preferably 6 months or less, further preferably 8 weeks or less, and most preferably 6 weeks or less, 5 weeks or less or 4 weeks or less.

When the composition is stored in the method according to the present embodiment, the composition is preferably stored as a packaged body accommodated in a container and tightly sealed in order to prevent volatilization of water. The packaged body may include a gas phase in the container, and the gas phase may be air. The packaged body including air as the gas phase is preferable because it can be produced at a low cost compared to a packaged body including an inert gas such as nitrogen as the gas phase.

### <Method for Controlling Oxidation of QH>

A second embodiment of the present invention relates to a method for controlling oxidation of QH, including storing a composition containing QH and having a water activity at 25°C of 0.50 or more.

According to the method according to the present embodiment, oxidation of QH can be efficiently controlled at a low cost.

Respective characteristics and preferred aspects of QH, the composition, and the step of storing the composition in the method according to the present embodiment are as described with respect to the method according to the first embodiment.

### <Composition>

A third embodiment of the present invention relates to a composition containing QH and having a water activity at 25°C of 0.50 or more.

Oxidation of QH in the form of the composition of the present embodiment is effectively controlled.

Respective characteristics and preferred aspects of QH and the composition in the composition according to the present embodiment are as described with respect to the method according to the first embodiment.

### Examples

### 1. Raw Materials

The present invention will be further specifically described with reference to the following Examples, but the present invention is not limited to these Examples. In Examples, reduced coenzyme Q10 (trade name: Kaneka QH) manufactured by KANEKA CORPORATION was used as a reduced coenzyme Q10 Form I crystal (QH Form I crystal).

### 2. Method for Evaluating Oxidation Stability

The weight ratio of reduced coenzyme Q10 to total coenzyme Q10 (namely, reduced coenzyme Q10/(oxidized coenzyme Q10 + reduced coenzyme Q10)) is defined as "QH ratio". The QH ratio was determined by the following HPLC analysis. For the evaluation of oxidation stability, a rate of the change in the QH ratio at the end of the evaluation from that at the beginning of the evaluation was defined as "QH residual rate", and the QH residual rate determined by the following expression was used as a measure of oxidation stability. QH residual rate (%) = 100 × QH ratio at the end of the evaluation/QH ratio at the beginning of the evaluation

### (HPLC Analysis Conditions)

Column: SYMMETRY C18 (manufactured by Waters); 250 mm (length), 4.6 mm (inner diameter)
Mobile phase: C₂H₅OH:CH₃OH = 4 : 3 (v : v)
Detection wavelength: 210 nm
Flow rate: 1 mL/min

### 3. Method for Measuring Water Activity

The water activity was measured with AquaLab Series 4TE (METER Group Inc.). A sample having a volume of about 5 ml in a sample dish was placed in AquaLab Series 4TE, and the water activity at 25°C was measured.

### 4. Example of Preparation of Reduced Coenzyme Q10 Form II Crystal (QH Form II Crystal)

To 611 g of ethanol, 89 g of the QH Form I crystal was added, and heated to 50°C to completely dissolve the QH Form I crystal. This solution was cooled, and 1.8 g of a reduced coenzyme Q10 Form II crystal prepared according to the description of Patent Literature 2 was added as a seed crystal when the temperature of the solution reached 36°C. The solution was cooled to 33.5°C over 7 hours, thereafter cooled to 25°C at a rate of 1°C/hour, and further cooled to 1°C at a rate of 10°C/hour to obtain a white slurry. The slurry obtained was filtered under reduced pressure to obtain a wet crystal, and the wet crystal was washed with cold ethanol and further dried under reduced pressure to obtain a QH Form II crystal.

### 5. Storage of QH Form II Crystal in High-Water Activity Composition

### [Example 1]

In a glass vial (volume 33 ml), 3 g of commercially available sandwich bread was placed. In the grass vial, 0.1 g of the QH Form II crystal obtained in Preparation Example was placed to be in contact with the sandwich bread, and the glass vial was sealed. This packaged body was stored under a temperature of 40°C and a relative humidity of 75% for 4 weeks, after which the QH residual rate was determined. The water activity at 25°C of a composition composed of 3 g of the sandwich bread and 0.1 g of the QH Form II crystal was 0.95.

### [Comparative Example 1]

In a glass vial (volume 33 ml), 0.1 g of the QH Form II crystal was placed. The glass vial was sealed, and stored under a temperature of 40°C and a relative humidity of 75% for 4 weeks, after which the QH residual rate was determined. The water activity at 25°C of the QH Form II crystal was 0.17.

The results obtained in Example 1 and Comparative Example 1 are summarized and shown in Table 1.

**[Table 1]**

| | Storage temperature (°C) | QH residual rate (%) |
|---|---|---|
| Example 1 | 40 | 94.7 |
| Comparative Example 1 | 40 | 87.5 |

### [Example 2]

A packaged body produced in the same manner as in Example 1 was stored under a temperature of 25°C and a relative humidity of 60% for 4 weeks, after which the QH residual rate was determined.

### [Example 3]

A packaged body containing the QH Form II crystal was produced by placing 0.1 g of the QH Form II crystal obtained in Preparation Example into an aluminum laminate bag (volume: about 1000 ml) and sealing the bag in a chamber with a controlled relative humidity of 50%. The water activity of the QH Form II crystal in the packaged body was here 0.50. Thereafter, the packaged body was stored at 25°C for 4 weeks, after which the QH residual rate was determined.

### [Example 4]

A packaged body was produced in the same manner as in Example 3 except that a chamber with a controlled relative humidity of 85% was used and the water activity of the QH Form II crystal was kept to be 0.85. The water activity of the QH Form II crystal in the packaged body was here 0.85. The packaged body was stored at 25°C for 4 weeks, after which the QH residual rate was determined.

The results obtained in Examples 2, 3, and 4 were summarized and shown in Table 2.

**[Table 2]**

| | Storage temperature (°C) | QH residual rate (%) |
|---|---|---|
| Example 2 | 25 | 97.1 |
| Example 3 | 25 | 92.3 |
| Example 4 | 25 | 95.3 |

Tables 1 and 2 show that a QH Form II crystal is stably stored in a composition having a water activity of 0.50 or more.

### 6. Storage of QH Form I Crystal in High-Water Activity Composition

### [Example 5]

In a glass vial (volume 33 ml), 3 g of commercially available sandwich bread was placed. In the grass vial, 0.1 g of the QH Form I crystal was placed to be in contact with the sandwich bread, and the glass vial was sealed. This packaged body was stored under a temperature of 25°C and a relative humidity of 60% for 4 weeks, after which the QH residual rate was determined. The water activity at 25°C of a composition composed of 3 g of the sandwich bread and 0.1 g of the QH Form I crystal was 0.95.

### [Example 6]

In a glass vial (volume 33 ml), 3 g of a 0.04% aqueous solution of hexaglycerin monolaurate and 0.1 g of the QH Form I crystal were placed, and mixed. The glass vial was sealed, and stored under a temperature of 25°C and a relative humidity of 60% for 4 weeks, after which the QH residual rate was determined. The water activity at 25°C of a composition composed of 3 g of the 0.04% aqueous solution of hexaglycerin monolaurate and 0.1 g of the QH Form I crystal was 1.00.

### [Comparative Example 2]

In a glass vial (volume 33 ml), 0.1 g of the QH Form I crystal was placed. The glass vial was sealed, and stored under a temperature of 25°C and a relative humidity of 60% for 4 weeks, after which the QH residual rate was determined. The water activity at 25°C of the QH Form I crystal was 0.13.

The results obtained in Examples 5 and 6, and Comparative Example 2 are summarized and shown in Table 3.

**[Table 3]**

| | Storage temperature (°C) | QH residual rate (%) |
|---|---|---|
| Example 5 | 25 | 84.0 |
| Example 6 | 25 | 61.5 |
| Comparative Example 2 | 25 | 33.3 |

### [Example 7]

A packaged body produced in the same manner as in Example 5 was stored under a temperature of 40°C and a relative humidity of 75% for 2 weeks, after which the QH residual rate was determined.

### [Example 8]

A packaged body produced in the same manner as in Example 6 was stored under a temperature of 40°C and a relative humidity of 75% for 2 weeks, after which the QH residual rate was determined.

The results obtained in Examples 7 and 8 are summarized and shown in Table 4.

**[Table 4]**

| | Storage temperature (°C) | QH residual rate (%) |
|---|---|---|
| Example 7 | 40 | 82.9 |
| Example 8 | 40 | 84.6 |

Tables 3 and 4 show that a QH Form I crystal is stably stored in a composition having a water activity of 0.50 or more as the QH Form II crystal used in Examples 1 to 4.

### 7. Storage of QH Dissolved in MCT Oil, in High-Water Activity composition

### [Example 9]

In a glass vial (volume 33 ml), 3 g of a 0.04% aqueous solution of hexaglycerin monolaurate were placed, and 1.5 g of MCT (medium-chain fatty acid triglyceride) containing 3.3% (w/w) of reduced coenzyme Q10 was additionally layered. The water activity at 25°C of a composition composed of 3 g of a 0.04% aqueous solution of hexaglycerin monolaurate and 1.5 g of MCT containing 3.3% (w/w) of reduced coenzyme Q10 was 0.98. The glass vial was sealed, and stored under a temperature of 25°C and a relative humidity of 60% for 4 weeks, after which the QH residual rate was measured and the QH residual rate was 96.2%.

It has been revealed from Example 9 that, even when a layer of an MCT solution containing a reduced coenzyme Q10 is in contact with water, reduced coenzyme Q10 present in the MCT solution is stably stored.

### 8. Method for Producing Co-crystal Composed of Reduced Coenzyme Q10 and Nicotinamide

To 7.85 g of ethanol, 4.33 g of the QH Form I crystal and 1.22 g of nicotinamide were added, and heated to 50°C, thereby completely dissolving the QH Form I crystal and nicotinamide. This solution was cooled, and the resulting white slurry was dried under reduced pressure, thereby obtaining a co-crystal composed of QH and nicotinamide. The melting point of the co-crystal obtained was measured with a differential scanning calorimeter (DSC200, manufactured by Hitachi, Ltd., heating rate: 1°C/min), and a peak was observed at a temperature different from the melting point (48°C) of the QH Form I crystal and the melting point (127°C) of nicotinamide, which were used as raw materials.

### [Example 10]

In a packaged body having a controlled internal relative humidity of 53%, 0.2 g of a co-crystal composed of QH and nicotinamide was enclosed in an opened state. The water activity of the co-crystal in the packaged body was here 0.53. The packaged body was stored at 40°C for 2 weeks, after which the QH residual rate was determined.

### [Comparative Example 3]

In a packaged body having a controlled internal relative humidity of 48%, 0.2 g of a co-crystal composed of QH and nicotinamide was enclosed in an opened state. The water activity of the co-crystal in the packaged body was here 0.48. The packaged body was stored at 40°C for 2 weeks, after which the QH residual rate was determined.

The results obtained in Example 10 and Comparative Example 3 are summarized and shown in Table 5.

**[Table 5]**

| | Storage temperature (°C) | QH residual rate (%) |
|---|---|---|
| Example 10 | 40 | 92.2 |
| Comparative Example 3 | 40 | 82.6 |

Table 5 shows that QH present in a co-crystal composed of QH and nicotinamide is also stably stored in a composition having a water activity of 0.50 or more.

All publications, patents and patent applications cited in the present description are incorporated herein by reference in their entirety.

The upper and/or lower limits of the numerical ranges described herein can be combined arbitrarily to define a preferred range. For example, a preferred range can be defined by arbitrarily combining the upper and lower limits of the numerical ranges, a preferred range can be defined by arbitrarily combining the upper limits of the numerical ranges, and a preferred range can be defined by arbitrarily combining the lower limits of the numerical ranges. The numerical range represented with the term "to" in the present application includes respective numerical values described before and after the term "to" as the upper and lower limits thereof.

It should be understood that throughout the present description, expressions in the singular forms include the concept of their plural forms unless otherwise specifically stated. Thus, articles in the singular forms (e.g., "a," "an," and "the" in the English language) should be understood to include the concept of their plural forms, unless otherwise specifically stated.

Although the embodiment of the present invention has been described in detail above, the specific configuration is not limited to this embodiment, and even when there are design changes within the scope of the present disclosure, they are included in the present disclosure.

## Claims

1. A method for storing reduced coenzyme Q10, comprising
storing a composition containing reduced coenzyme Q10 and having a water activity at 25°C of 0.50 or more.

2. The method according to claim 1, wherein the composition is a mixed composition in which the reduced coenzyme Q10 and one or more other components are mixed.

3. The method according to claim 1, wherein the composition is a multiphase composition including a first phase containing the reduced coenzyme Q10 and a second phase containing one or more other components, the first and second phases being in contact with each other.

4. The method according to any one of claims 1 to 3, wherein the reduced coenzyme Q10 is one or more selected from the group consisting of a reduced coenzyme Q10 Form I crystal, a reduced coenzyme Q10 Form II crystal, a co-crystal composed of reduced coenzyme Q10 and one or more other compounds, an amorphous solid of reduced coenzyme Q10, and a composition in which reduced coenzyme Q10 is dissolved in a solvent and/or a fat-soluble material.

5. The method according to any one of claims 1 to 4, wherein the composition includes a substance releasing water.

6. A method for controlling oxidation of reduced coenzyme Q10, comprising
storing a composition containing reduced coenzyme Q10 and having a water activity at 25°C of 0.50 or more.

7. The method according to claim 6, wherein the composition is a mixed composition in which the reduced coenzyme Q10 and one or more other components are mixed.

8. The method according to claim 6, wherein the composition is a multiphase composition including a first phase containing the reduced coenzyme Q10 and a second phase containing one or more other components, the first and second phases being in contact with each other.

9. The method according to any one of claims 6 to 8, wherein the reduced coenzyme Q10 is one or more selected from the group consisting of a reduced coenzyme Q10 Form I crystal, a reduced coenzyme Q10 Form II crystal, a co-crystal composed of reduced coenzyme Q10 and one or more other compounds, an amorphous solid of reduced coenzyme Q10, and a composition in which reduced coenzyme Q10 is dissolved in a solvent and/or a fat-soluble material.

10. The method according to any one of claims 6 to 9, wherein the composition includes a substance releasing water.

11. A composition comprising reduced coenzyme Q10 and having a water activity at 25°C of 0.50 or more.

12. The composition according to claim 11, wherein the composition is a mixed composition in which the reduced coenzyme Q10 and one or more other components are mixed.

13. The composition according to claim 11, wherein the composition is a multiphase composition including a first phase containing the reduced coenzyme Q10 and a second phase containing one or more other components, the first and second phases being in contact with each other.

14. The composition according to any one of claims 11 to 13, comprising a substance releasing water.
